# FASCICULE DE BREVET EUROPEEN

(11) **EP 0 653 195 B1**
(45) Date de publication et mention de la délivrance du brevet: **17.02.1999**
(21) Numéro de dépôt: 94402503.0
(22) Date de dépôt: 07.11.1994
(51) Int. Cl.: A61F 2/42, A61F 2/30

(54) **Prothèse articulaire, en particulier pour articulations phalangiennes**
Gelenkprothese, inbesondere für Finger- oder Zehen-Gelenke
Joint prothesis, especially for phalangeal prosteses

(30) Priorité: 08.11.1993 FR 9313250
(43) Date de publication de la demande: 17.05.1995
(73) Titulaire: COMMISSARIAT A L'ENERGIE ATOMIQUE, 75015 Paris (FR)
(72) Inventeur: Gaidry, Jacques, F-38320 Eybens (FR); Ranc, René, F-38100 Grenoble (FR); Moutet, François, F-38240 Meylan (FR)
(74) Mandataire: Dubois-Chabert, Guy

(56) Documents cités:
- EP-A- 0 169 976
- EP-A- 0 278 184
- EP-A- 0 280 424
- FR-A- 2 245 329
- FR-A- 2 356 406
- FR-A- 2 417 292
- FR-A- 2 680 967

## Description

### Domaine de l'invention

L'invention concerne les prothèses articulaires et en particulier les prothèses digitales destinées à remplacer des articulations métacarpophalangiennes et/ou interphalangiennes.

### Art antérieur et problème posé

Diverses maladies, telles que l'arthrose post-traumatique, la polyarthrite évolutive, l'arthrite septique et certains états rhumatismaux conduisent à la détérioration progressive, voire à la destruction, des articulations digitales. En conséquence, depuis un grand nombre d'années, on utilise des prothèses ou implants articulaires de diverses natures pour tenter de restaurer l'usage des doigts de la main chez les patients atteints de telles maladies.

La destruction de certaines articulations interphalangiennes proximales (IPP) et métacarpophalangiennes (MP) des doigts longs pose au chirurgien de la main un problème thérapeutique encore incomplètement résolu. Par exemple, la récupération d'amplitudes fonctionnelles nécessite le remplacement des surfaces articulaires, que la destruction articulaire de ces surfaces soit la conséquence d'une arthrose post-traumatique, de l'évolution d'une polyarthrite évolutive ou d'une arthrite septique.

Les concepts d'arthroplastie possibles à l'heure actuelle sont : les arthroplasties contraintes d'une part, où les deux parties articulées sont mécaniquement liées d'une façon définitive, et les arthroplasties semi-contraintes ou non-contraintes de l'autre, où les deux pièces sont prévues avec une liaison mécanique non permanente pour les premières et sans liaison pour les secondes. Dans ces deux dernières catégories, les problèmes de stabilité latérale ont conduit les spécialistes à proposer un implant contraint à charnière ne possédant qu'un seul degré de liberté. Par exemple, on peut citer le document FR-A-2 680 967, déposé par le présent demandeur.

En référence à la figure 1, ce document décrit un implant composé des éléments suivants :
- une chape femelle 1 solidaire d'une première pièce diaphysaire 7 munie de deux orifices alignés et transversaux par rapport à cette première pièce ;
- une chape mâle 3, munie d'un orifice transversal par rapport à une deuxième pièce diaphysaire 9, coopérant avec la chape femelle 1 et solidaire de cette deuxième pièce diaphysaire 9 ;
- un axe 6 monté en force dans les deux orifices de la chape femelle 1 et s'étendant au travers de l'orifice de la chape mâle 3 ;
- une bague 4 entourant l'axe 6 et engagée en force dans l'orifice de la chape mâle 3 ;
- deux flasques latéraux 2 pouvant être insérés dans des encoches 1A de la chape femelle 1 munies d'une découpure carrée, cette découpure étant destinée à recevoir des joues 5 de maintien en place de la bague 4.

De plus, il faut que la bague 4 émerge de chaque côté de l'orifice de la chape mâle 3 et que l'épaisseur de chaque joue 5 soit supérieure à celle des flasques latéraux 2.

On note qu'il est composé d'un nombre élevé de pièces constitutives, à savoir huit, dont quatre en titane et quatre en carbone. Or, toute réduction du nombre de pièces entraîne des problèmes d'usinage consécutifs à l'utilisation de l'électro-érosion. Par contre, une réduction du nombre de pièce entraîne une simplification de la conception et du montage de l'ensemble. En effet, la multiplication des pièces à monter dans des différentes conditions d'assemblage, ajustement glissant ou serré, impose des tolérances de fabrication très serrées dans la mesure où elles s'ajoutent et exigent des usinages précis et donc onéreux.

De plus, le concept de cette articulation ne permet pas d'envisager son évolution vers un système moins rigide, plus proche de certaines articulations naturelles.

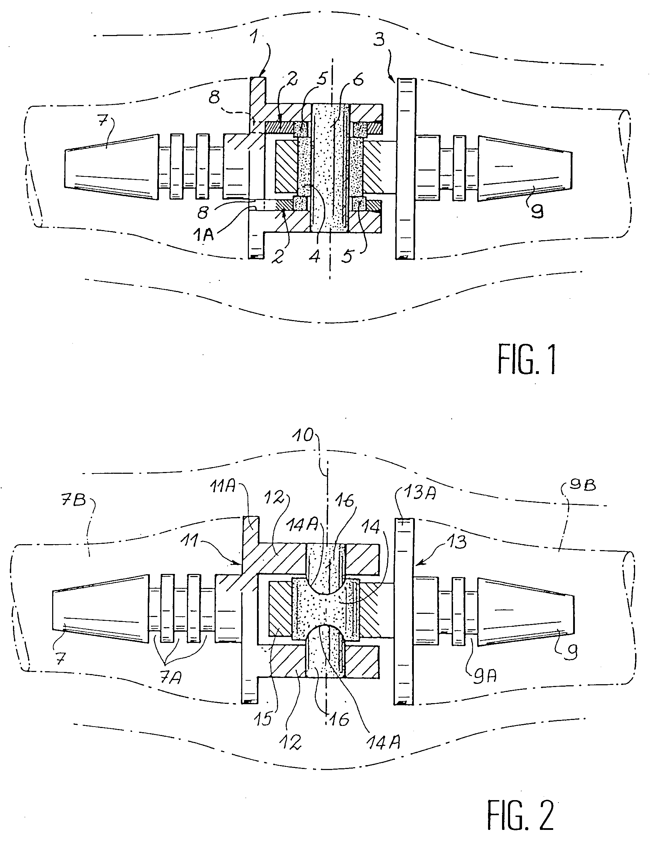

En référence à la figure 2, une autre prothèse articulaire selon l'art antérieur reprend le schéma général de celle de l'art antérieur, décrite en regard de la figure 1. On y retrouve une première pièce 11 comportant une chape femelle 12 et une pièce diaphysaire 7 implantée dans un des os 7B d'une phalange ou d'un métacarpe. De manière similaire, on retrouve une deuxième pièce 13 comportant une chape mâle 15 et une pièce diaphysaire 9 implantée dans l'os 9B d'une phalange. Ceci est imposé par la procédure de montage. La chape mâle est toujours implantée dans une phalange. La pièce diaphysaire 7 a été représentée équipée de trois gorges 7A destinées à faciliter l'implantation de cette première pièce 11 par sa partie diaphysaire 7 dans l'os 7B. De même, la deuxième pièce 13 est équipée d'au moins une gorge 9A, pratiquée dans sa partie diaphysaire 9 pour faciliter son implantation dans l'os 9B.

Comme pour l'articulation de la figure 1, le mouvement des deux parties phalangiennes s'effectue autour de l'axe de rotation 10 qui est en fait le plus près possible de l'axe naturel d'une articulation phalangienne. La chape femelle 12 de la première pièce 11 est du même type que celle décrite précédemment, c'est-à-dire qu'elle comprend deux branches latérales percée chacune d'un trou centré autour de l'axe 10 de l'articulation. En concordance, la deuxième pièce 13 comporte une chape mâle 15 comportant un trou transversal devant être placé coaxialement avec l'axe 10 de l'articulation.

On réalise la liaison mécanique entre les branches de la chape femelle 12 et la chape mâle 15 au moyen de deux demi-axes 16 solidaires des deux branches et par une pièce d'appui 14 solidaire de la chape mâle 15. Les deux demi-axes 16 sont emmanchés en force chacun dans le trou d'une branche de la chape femelle 12 de manière à être fixés définitivement dans celle-ci. Ils comportent chacun une extrémité arrondie placée l'une en regard de l'autre. Ces demi-axes 16 sont centrés l'un par rapport à l'autre et symétriques par rapport à l'axe longitudinal de la prothèse. De même, la pièce d'appui 14 est emmanchée en force dans le trou de la chape mâle 15 pour y être fixée définitivement. Elle comporte deux cavités latérales concaves 14A dans lesquelles sont placées les extrémités sphériques des deux demi-axes 16.

On comprend ainsi que la liaison entre les deux pièces 11 et 13 se fait par une articulation à un degré de liberté par rotation autour de l'axe 10 de cette articulation défini par les trous de la chape femelle 12 et de la chape mâle 15. Bien entendu, dans ce cas, les rayons des extrémités sphériques des deux demi-axes 16 et des cavités concaves 14A de la pièce d'appui 14 sont égaux. De plus, la largeur de la pièce d'appui 14 doit être inférieure à la distance séparant les deux branches de la chape femelle.

On note que la première pièce 11 et la deuxième 13 comportent chacune un épaulement 11A et 13A permettant de choisir, lors de la fabrication en fonction de la taille de la prothèse, la distance de l'axe d'articulation 10 par rapport aux os 7B et 9B des phalanges ou du métacarpe.

Il est préférable de réaliser les chapes femelle et mâle en un alliage de titane de qualité médicale, par exemple le Ti.6Al.4V.

Les demi-axes sont réalisés en graphite entièrement revêtus d'une couche de pyrocarbone de forte épaisseur (au moins de cent micromètres) par un procédé de dépôt chimique en phase vapeur à température très élevée (1 400°C).

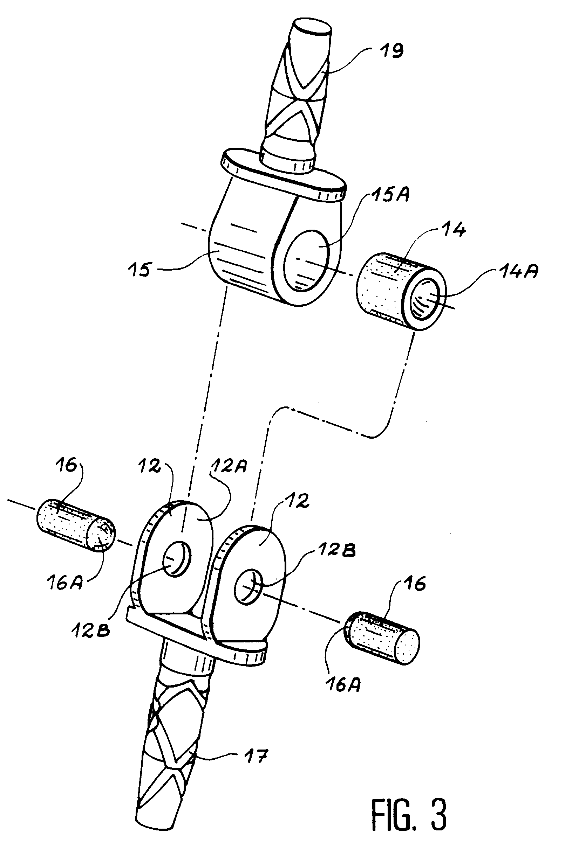

En référence à la figure 3, le montage d'une telle prothèse peut être le suivant.

La pièce d'appui 14 est d'abord introduite en force dans l'alésage 15A de la chape mâle 15, sa longueur étant de préférence supérieure à celle de l'alésage 15A. Le dépassement de cette pièce d'appui 14 est réparti également de part et d'autre des deux faces de la chape mâle 15.

Les deux demi-axes 16 sont ensuite emmanchés en force dans les branches de la chape femelle, leurs extrémités sphériques 16A étant orientées l'une vers l'autre. Dans un premier temps, ces dernières ne dépassent pas le niveau de la face intérieure 12A des branches de la chape femelle.

Ensuite, comme le suggèrent les traits mixtes de la figure 3, la chape mâle est introduite entre les branches de la chape femelle 12, les surfaces concaves sphériques 14A étant placées en regard des orifices 12B de la chape femelle, et en particulier des surfaces sphériques 16A des demi-axes 16.

Enfin, ces derniers sont enfoncés à la presse de manière à ce que chacune des extrémités sphériques 16A dépasse symétriquement des faces intérieures 12A des branches de la chape femelle 12, pour occuper toute la surface concave 14A correspondante de la pièce d'appui 14.

Des gorges croisées 17 et 19 ont été usinées sur les parties diaphysaires des deux pièces pour améliorer leur fixation dans les os.

### Résumé de l'invention

A cet effet, l'objet principal de l'invention est une prothèse articulaire, de préférence digitale pour articulations métacarpophalangiennes et interphalangiennes, comprenant deux pièces implantables dans les os de l'articulation (des phalanges et/ou du métacarpe) et formant une articulation pivotante. Cette prothèse comprend :
- une première pièce constituée d'une chape femelle, munie de deux orifices alignés, et solidaire d'une première partie diaphysaire ;
- une deuxième pièce, comprenant une chape mâle, coopérant avec la chape femelle, et solidaire d'une deuxième partie diaphysaire ;
- deux demi-axes montés en force dans les orifices de cette chape femelle, les deux extrémités de ces deux demi-axes en regard l'une de l'autre sont sphériques, la chape mâle ayant deux faces latérales opposées concaves pour recevoir chacune une desdites extrémités en regard l'une de l'autre, l'une au moins de ces deux faces latérales étant sphérique et de même rayon que celui de l'extrémité du demi-axe correspondant.

Selon l'invention, le diamètre du demi-axe correspondant à la face concave sphérique est supérieur au diamètre du demi-axe correspondant à la deuxième face permettant le ou les mouvements de translation.

Dans la réalisation préférentielle de la chape mâle, les deux faces latérales opposées sont réalisées dans une même pièce d'appui cylindrique emmanchée à force dans l'orifice de la chape mâle.

Dans une première variante de réalisation, la chape mâle a une première face latérale concave sphérique et une deuxième face latérale concave ayant la forme de l'enveloppe d'un mouvement de translation effectué par l'extrémité sphérique du demi-axe correspondant.

Dans une deuxième variante, la chape mâle a une première face latérale concave sphérique et une deuxième face latérale concave dont la forme est l'enveloppe de deux mouvements de translation perpendiculaire effectués par l'extrémité sphérique du demi-axe correspondant. Ceci multiplie les possibilités de débattement de la prothèse.

Dans une réalisation préférentielle, la chape mâle peut être complétée d'au moins une butée d'arrêt en rotation faisant saillie pour venir contre la surface de la chape femelle lors d'une rotation de l'articulation.

### Liste des figures

Plusieurs réalisations de l'invention et de ses caractéristiques techniques sont maintenant décrites à l'aide des quelques figures représentant respectivement :
- figure 1, en coupe partielle, une première prothèse articulaire selon l'art antérieur ;
- figure 2, en coupe partielle, une deuxième prothèse articulaire selon l'art antérieur ;
- figure 3, en vue éclatée, une réalisation de la prothèse décrite à la figure 2 ;
- figure 4, en coupe partielle, une réalisation principale de l'articulation de la prothèse selon l'invention ;
- figure 5, un schéma relatif au mouvement complémentaire obtenu avec l'articulation de la figure 4 ;
- figure 6, en vue cavalière, une vue explicative de deux mouvements complémentaires obtenus avec l'articulation de la figure 4 ;
- figure 7, en coupe partielle, une réalisation particulière de l'invention pour une articulation phalangienne ;

### Description détaillée des caractéristiques nouvelles de l'invention

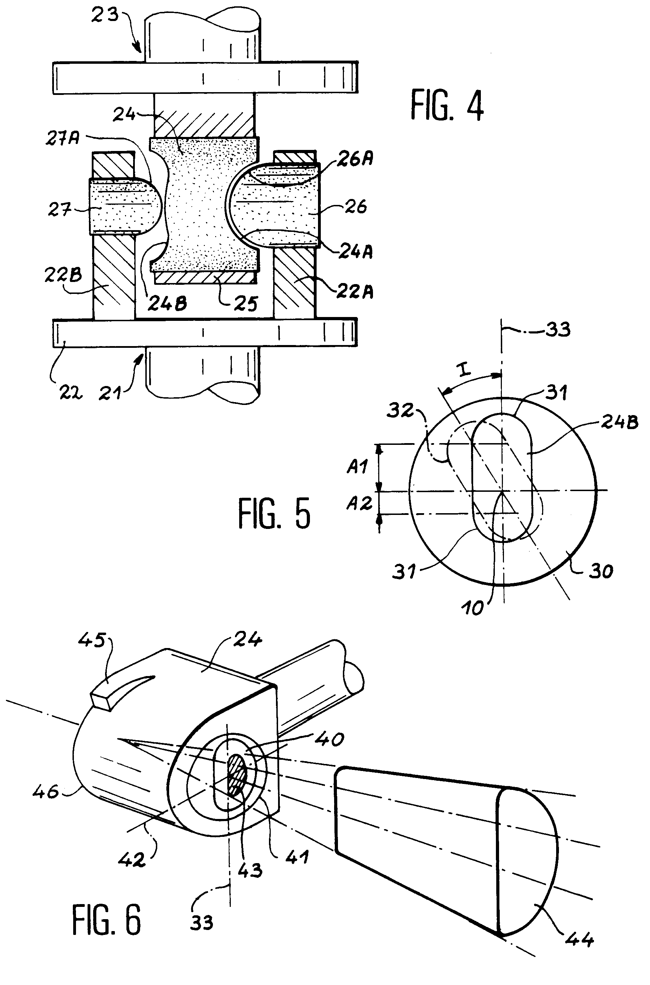

En référence à la figure 4, l'articulation selon l'invention prévoit une possibilité supplémentaire de mouvement relatif de la première pièce 21 par rapport à la deuxième 23. En d'autres termes, on permet une possibilité supplémentaire de mouvement pour le doigt sur lequel est montée la prothèse articulaire selon l'invention. En effet, dans cette dernière, la pièce d'appui 24 possède deux faces latérales concaves 24A et 24B différentes. En correspondance, les deux demi-axes 26 et 27 sont de dimensions différentes. Un premier demi-axe 26 est cylindrique et de grand diamètre. Il est logé dans un orifice correspondant d'une première branche 22A de la chape femelle. Sa surface sphérique 26A est donc d'un rayon plus important que celle 27A du deuxième demi-axe 27 logé dans une deuxième branche 22B de la chape femelle.

En correspondance, la pièce d'appui 24 possède une première surface concave 24A sphérique d'un diamètre égal à celui de la surface sphérique 26A du premier demi-axe 26. De l'autre côté, la pièce d'appui 24 possède une surface creuse 24B destinée à recevoir l'extrémité 27A du deuxième demi-axe 27. Or, cette cavité 24B possède une partie centrale convexe et des extrémités concaves. Le centre de cette partie convexe est confondu avec celui de l'extrémité sphérique 26A du demi-axe 26. Elle permet un mouvement de rotation de la pièce d'appui 24 autour du centre de la sphère formée par la surface sphérique 26A du premier demi-axe 26. De cette manière, l'extrémité de la surface sphérique 27A tangente toute la partie convexe de la cavité 24B qui est placée en regard d'elle. Dans la position extrême de ce mouvement, la surface sphérique 27A du deuxième demi-axe 27 se trouve en contact avec l'une des parties concaves latérales de la cavité 24B.

La figure 5 permet de visualiser la possibilité de déplacement supplémentaire offerte par la réalisation de la figure 4. Elle illustre la possibilité de choisir l'orientation du déplacement supplémentaire en inclinant la rainure 31 par rapport à l'axe vertical 33. En effet, la surface circulaire 30 représente la pièce d'appui 24 vue du côté gauche, la chape femelle et le demi-axe correspondant 27 étant enlevés. La cavité 24B affecte donc la forme d'une rainure 31 dont les extrémités sont sphériques, en correspondance avec le rayon de la surface sphérique 27A du demi-axe correspondant 27. Les amplitudes respectives A1 et A2 de part et d'autre du centre de la pièce d'appui 24, c'est-à-dire au niveau de l'axe d'articulation 10 sont choisies en fonction des débattements angulaires latéraux que l'on désire donner aux doigts bénéficiaires de la prothèse articulaire dont il est question sur cette figure.

La rainure 32 représentée en traits mixtes est inclinée par rapport à l'axe vertical 33 de cette figure. Ceci signifie que la rotation du doigt autour de son axe est permise dans un débattement correspondant au produit de l'angle I d'inclinaison de cette rainure et de la longueur A1 correspondante.

On comprend donc qu'il est facile d'obtenir à partir d'un mouvement de translation supplémentaire au niveau de l'articulation de nombreuses possibilités de mouvements pour les doigts bénéficiant de cette prothèse articulaire.

La figure 6 montre la forme de la cavité 40 de la pièce d'appui 24 selon l'invention. Celle-ci affecte en effet sur un côté la forme de la cavité 24B des figures 4 et 5. Ceci veut dire qu'un débattement selon une direction 42 perpendiculaire à la direction 33 évoquée aux figures 4 et 5 est possible. Ceci se traduit par un débattement selon deux directions perpendiculaires 33 et 42 du demi-axe correspondant. La zone hachurée 43 représente tous les endroits où peut se trouver le centre du demi-axe 27 au fond de sa cavité 40.

Le volume angulaire de débattement du doigt, mis à part l'articulation autour de l'axe 10, est symbolisé par la forme 44 placée sur la partie droite de la figure 6.

Dans cet exemple, les côtés gauches de la zone hachurée 43 et de la forme 44 sont en partie droits, car la cavité 24B possède elle-même un côté gauche qui est droit. Ceci est prévu pour tenter de reproduire tous les mouvements précis d'une articulation particulière du corps humain, par exemple métacarpophalangienne, et leurs limitations.

Dans ce cas, le doigt pourra bénéficier de la rotation initiale autour de l'axe 10, d'une rotation autour d'un axe de rotation perpendiculaire à l'axe 10 correspondant à un écartement des doigts les uns par rapport aux autres et d'une rotation encore perpendiculaire autour de l'axe du doigt. En d'autres termes, la phalange possède par rapport à une autre phalange ou à un métacarpe trois degrés de liberté, dans un débattement limité par une butée 45 et la forme de la cavité 40. En effet, la butée d'arrêt 45 peut être prévue sur le côté de la pièce d'appui 24 de manière à venir en saillie par rapport à la surface externe 46 de celle-ci et venir buter contre une surface interne de la chape femelle, dans le but de limiter la rotation autour de l'axe 10.

L'application de l'invention est, à première vue, limitée aux articulations métacarpophalangiennes des doigts longs, les seuls à posséder une mobilité latérale.

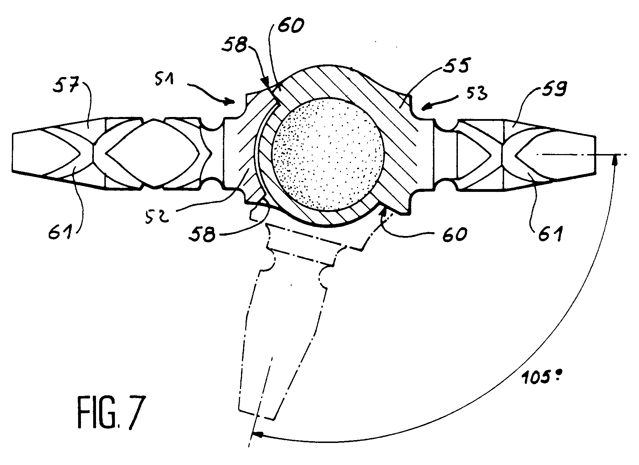

Cependant, la figure 7 représente une application de la prothèse articulaire en remplacement d'une charnière digitale. On retrouve sur cette figure tous les éléments de l'invention représentée par la figure 4, pas avec exactement les mêmes formes. La grande différence est que tous les espaces existants entre les éléments de la réalisation de la figure 4 sont réduits au minimum. Les espaces libres ménagés entre les éléments en mouvement étant réduits, on évite une réponse trop importante de la part du tissu qui pourrait se traduire par une expansion de celui-ci et gêner la fonction de l'articulation jusqu'à provoquer l'immobilisation de celle-ci.

On peut constater la présence de butées 60 sur la partie mâle 55. En correspondance, se trouvent également deux butées 58 sur la base des branches de la chape femelle 52. L'agencement des butées 60 et 58, en partie intégré à l'enveloppe extérieure de la prothèse, les rend ainsi moins agressives vis-à-vis de l'appareil extenseur pendant les mouvements de rotation de la prothèse.

On note que les plans de contact entre les butées 60 et 58 sont orientés vers le centre de la rotation. Ceci supprime l'usure génératrice et d'éventuels débris très préjudiciables au fonctionnement de l'ensemble.

Cette prothèse est également représentée en traits mixtes dans une position repliée, la rotation autour de l'axe 10 de la figure 7 pouvant atteindre un débattement de 105°.

On a également représenté sur cette figure 7 des gorges croisées 61 à pas très longs sur les parties diaphysaires 57 et 59 correspondant respectivement aux parties femelle 51 et mâle 53. On obtient ainsi une amélioration de la liaison de celles-ci avec le ciment d'implantation puisque la longueur totale de contact, et donc la surface d'adhérence, est supérieure. Ceci favorise également le maintien de l'adhérence du ciment sur toute la surface des gorges 61 pendant la mise en place de la prothèse. Ceci est particulièrement efficace lors de l'introduction de la queue dans le canal médulaire, ce mouvement provoquant un glissement de ciment plutôt qu'un bourrage avec un risque de perte d'adhérence. C'est également le cas lors du changement de position avant le durcissement définitif du ciment. En effet, l'implantation n'est possible dans ce cas que lorsque l'articulation est orientée à cent cinq degrés, car seule cette position permet d'introduire les queues dans les canaux correspondants. Or, c'est en position alignée qu'a lieu la prise du ciment et lors de cette rotation les pièces diaphysaires 57, 59 sont amenées à faire une combinaison de rotation et translation par rapport aux phalanges.

### Avantage principal de l'invention

Le principe de l'articulation à partir de zones en pivotement sphérique permet de proposer des charnières contraintes avec au moins un degré de liberté latéral en plus de la rotation, ce qui représente mieux le fonctionnement réel de l'articulation métacarpophalangienne naturelle des doigts longs, ceci sans compter le degré de liberté apporté par la forme allongée de la cavité 24B.

## Revendications

1. Prothèse articulaire pour articulations comprenant deux pièces (11, 13, 21, 23, 51, 53) implantables dans les os (7B, 9B) de l'articulation et formant une articulation pivotante, la prothèse comprenant :
- une première pièce constituée d'une chape femelle (12, 22, 52) munie de deux orifices (12B) alignés et solidaire d'une première partie diaphysaire (7, 57) ;
- une deuxième pièce constituée d'une chape mâle (15, 25, 55) coopérant avec la chape femelle et solidaire d'une deuxième partie diaphysaire (9, 59),
- deux demi-axes (16, 26, 27) montés en force dans les orifices (12B) de cette chape femelle, les deux extrémités (16A, 26A, 27A) placées en regard l'une de l'autre sont sphériques,
la chape mâle ayant deux faces latérales opposées concaves (24A, 24B) pour recevoir chacune une desdites extrémités en regard et dont une première face latérale (24A) est sphérique et de même rayon que celui de l'extrémité du demi-axe correspondant (16, 26),
caractérisée en ce que le diamètre du demi-axe (26) correspondant à la première face concave sphérique (24A) est supérieur au diamètre du demi-axe (27) correspondant à la deuxième face (24B) permettant au moins un mouvement de translation.

2. Prothèse articulaire selon la revendication 1, caractérisée en ce que les deux faces latérales opposées de la chape mâle (25, 55) sont réalisées dans une pièce d'appui cylindrique (24) emmanchée en force dans un orifice de la chape mâle (25, 55).

3. Prothèse articulaire selon la revendication 1, caractérisée en ce que la chape mâle (25, 55) a une première face latérale concave sphérique (24A) et une deuxième face latérale concave (24B) dont la forme est l'enveloppe d'un mouvement de translation de l'extrémité sphérique (27A) du demi-axe correspondant (27).

4. Prothèse articulaire selon la revendication 1, caractérisée en ce que la chape mâle (25, 55) a une première face latérale concave sphérique (24A) et une deuxième face latérale concave dont la forme est l'enveloppe de deux mouvements de translation perpendiculaires de l'extrémité sphérique du demi-axe correspondant.

5. Prothèse articulaire selon la revendication 1, caractérisée en ce que la chape mâle (25, 55) possède au moins une butée d'arrêt (45, 60) d'arrêt en rotation faisant saillie pour venir contre une surface (58) de la chape femelle lors d'une rotation de l'articulation.

6. Prothèse articulaire selon la revendication 1, utilisée pour une articulation digitale, métacarpophalangienne ou interphalangienne, caractérisée en ce qu'elle comprend des gorges croisées (61) sur les parties diaphysaires (57, 59) pour faciliter l'implantation de la prothèse dans les os.

## Claims

1. Joint prosthesis comprising two components (11, 13, 21, 23, 51, 53) which can be implanted in the bones (7B, 9B) of the joint and forming a pivot joint, the prosthesis comprising:
- a first component consisting of a female bracket (12, 22, 52) provided with two aligned orifices (12B) and integral with a first diaphysial part (7, 57);
- a second component consisting of a male bracket (15, 25, 55) cooperating with the female bracket and integral with a second diaphysial part (9, 59),
- two half-pins (16, 26, 27) mounted by force in the orifices (12B) of this female bracket, the two ends (16A, 26A, 27A) placed facing one another are spherical,
- the male bracket having two opposite concave side faces (24A, 24B) for each receiving one of the said facing ends, and of which a first side face (24A) is spherical and of the same radius as that of the end of the corresponding half-pin (16, 26),
characterized in that the diameter of the half-pin (26) corresponding to the first spherical concave face (24A) is greater than the diameter of the half-pin (27) corresponding to the second face (24B) permitting at least one movement of translation.

2. Joint prosthesis according to Claim 1, characterized in that the two opposite side faces of the male bracket (25, 55) are formed in a cylindrical bearing component (24) engaged by force in an orifice of the male bracket (25, 55).

3. Joint prosthesis according to Claim 1, characterized in that the male bracket (25, 55) has a first spherical concave side face (24A) and a second concave side face (24B), of which the shape is the envelope of a movement of translation of the spherical end (27A) of the corresponding half-pin (27).

4. Joint prosthesis according to Claim 1, characterized in that the male bracket (25, 55) has a first spherical concave side face (24A) and a second concave side face, of which the shape is the envelope of two perpendicular movements of translation of the spherical end of the corresponding half-pin.

5. Joint prosthesis according to Claim 1, characterized in that the male bracket (25, 55) has at least one projecting rotational limit stop (45, 60) which abuts against a surface (58) of the female bracket during rotation of the joint.

6. Joint prosthesis according to Claim 1, used for a finger joint, either metacarpo-phalangeal or interphalangeal, characterized in that it comprises crossed grooves (61) on the diaphysial parts (57, 59) in order to facilitate implantation of the prosthesis in the bones.

## Patentansprüche

1. Gelenkprothese für Gelenke, zwei Teile (11, 13, 21, 23, 51, 53) umfassend, die in die Knochen (7B, 9B) des Gelenks implantiert werden können und dabei ein schwenkbares Gelenk bilden, wobei die Prothese umfaßt:
- ein erstes Teil, gebildet durch eine Gelenkgabel (12, 22, 52), versehen mit zwei fluchtenden Durchgangslöchern (12B) und fest verbunden mit einem diaphysischen Teil (7, 57);
- ein zweites Teil, gebildet durch einen Gelenkkopf (15, 25, 55), mit der Gelenkgabel zusammenwirkend und fest verbunden mit einem zweiten diaphysischen Teil (9, 59),
- zwei Halbachsen (16, 26, 27) mit Preßsitz in den Durchgangslöchern (12B) dieses Gabelgelenks, wobei die beiden sich gegenüberstehenden Enden (16A, 26A, 27A) kugelförmig sind und der Gelenkkopf zwei konkave entgegengesetzte Seitenflächen (24A, 24B) aufweist, von denen jede einem der genannten gegenüberstehenden Enden als Aufnahme dient und dabei eine erste Seitenfläche (24A) kugelförmig ist und denselben Radius aufweist wie das Ende der entsprechenden Halbachse (16, 26)
**dadurch gekennzeichnet,**
daß der Durchmesser der der ersten, kugelförmig konkaven Fläche (24A) entsprechenden Halbachse (26) größer ist als der wenigstens eine Translationsbewegung ermöglichende Durchmesser der der zweiten Fläche (24B) entsprechenden Halbachse (27).

2. Gelenkprothese nach Anspruch 1, dadurch gekennzeichnet, daß die beiden entgegengesetzten Seitenflächen des Gelenkkopfs (25, 55) in einem zylindrischen Abstützstück (24) realisiert sind, das in ein Durchgangsloch des Gelenkkopfs (25, 55) eingepreßt ist.

3. Gelenkprothese nach Anspruch 1, dadurch gekennzeichnet, daß der Gelenkkopf (25, 55) eine erste, kugelförmig konkave Seitenfläche (24A) und eine zweite, konkave Seitenfläche (24B) aufweist, deren Form die Hüllfläche einer Translationsbewegung des kugelförmigen Endes (27A) der entsprechenden Halbachse (27) ist.

4. Gelenkprothese nach Anspruch 1, dadurch gekennzeichnet, daß der Gelenkkopf (25, 55) eine erste, kugelförmig konkave Seitenfläche (24A) und eine zweite, konkave Seitenfläche aufweist, deren Form die Hüllfläche von zwei zueinander senkrechten Translationsbewegungen des kugelförmigen Endes der entsprechenden Halbachse ist.

5. Gelenkprothese nach Anspruch 1, dadurch gekennzeichnet, daß der Gelenkkopf (25, 55) wenigstens einen vorstehenden Endanschlag (45, 60) zum Anhalten der Drehbewegung aufweist, der bei einer Drehung des Gelenks gegen eine Fläche (58) der Gelenkgabel stößt.

6. Gelenkprothese nach Anspruch 1, benutzt für ein Finger-, Mittelhand- oder Zehengelenk, dadurch gekennzeichnet, daß sie in den diaphysischen Teilen (57, 59) überkreuzte Rillen (61) umfaßt, um die Implantation der Prothese in die Knochen zu erleichtern.
